# EUROPEAN PATENT SPECIFICATION

(11) **EP 4 173 645 B1**
(45) Date of publication and mention of the grant of the patent: **02.07.2025**
(21) Application number: 22205042.9
(22) Date of filing: 02.11.2022
(51) Int. Cl.: A61L 9/12

(54) **AIR FRESHENER WITH STEM STRUCTURE WITH STOPS**
ASSAINISSEUR D'AIR AVEC STRUCTURE À TIGE AVEC ARRÊTS
LUFTERFRISCHER MIT STIELSTRUKTUR MIT ANSCHLÄGEN

(30) Priority: 02.11.2021 ES 202132143 U
(43) Date of publication of application: 03.05.2023
(73) Proprietor: ZENIT Estudio de Diseño e Innovación S.L., 46010 Valencia (ES)
(72) Inventor: Blasco Feo, Vicente, Valencia (ES)
(74) Representative: Soler Lerma, Santiago

(56) References cited:
- CN-U- 209 417 532
- CN-U- 211 912 308
- CN-U- 213 884 389
- JP-A- 2002 011 086
- US-A1- 2006 231 641
- US-A1- 2020 114 035

## Description

The present invention relates to an air freshener having a structure comprising a stem with two stops, at least one of them removable, so that an impregnated element of the substance to be evaporated can be inserted into the stem, as well as, the head of a clamp that will fasten the assembly to, for example, the ventilation grid of an automobile.

In this way, a small, compact, versatile air freshener is achieved, which can combine fragrances depending on whether one or more impregnated elements are inserted and which can be used in the automotive field and elsewhere.

It belongs is to the field of air fresheners.

### BACKGROUND OF THE INVENTION

Air fresheners are known which comprise an impregnated element which, by the action of the air, evaporates the impregnated substance.

Occasionally such evaporation is forced by arranging the impregnated element in the air stream of, for example, the ventilation system of a vehicle.

Several examples of air fresheners that are arranged in the air stream of the ventilation system of a vehicle can be found in the patent literature.

Patent EP1992365 relates to a housing comprising a rear and a front part, suitable for arranging a reservoir with perfume therein and wherein the parts of that housing can rotate relative to each other by varying the air flow through the device.

Utility model ES1163433U refers to an air freshener comprising a container with a porous plug in such a way that said plug, initially dry, is impregnated with the substance present in the container and, as it is placed in the air stream of the ventilation system, evaporation is forced.

Utility model ES1054958U relates to a cage with a fixed and a folding part where the fixed part is attached to the ventilation grid of the vehicle while the folding part can pivot to facilitate access to the interior of the cage for the purpose of replacing the tablet of impregnated material.

Also, patent KR101661882 refers to a box containing a material to be evaporated which is arranged in the air stream of the vehicle ventilation system.

Patent KR20170031579 relates to a device that presents a blister type replacement with a plurality of cavities, in each of which is housed a tablet, preferably spherical, of material impregnated in such a way that the user, pressing the cavities of the blister and breaking the seal activates the number of tablets desired. The blister is arranged in a support that is installed in the air stream of the car's ventilation system.

Patent US 2020114035 relates to a fragrance diffuser has a diffuser element connecting a first reservoir to a second reservoir. As fragrant oil flows between the reservoirs, the diffuser element becomes saturated with oil and diffuses the fragrance or scent.

Patent US2006231641 relates to delivery systems for emitting or releasing volatile materials to the atmosphere wherein said delivery system comprises at least one container comprising at least one fluid reservoir; at least one evaporative surface device opening located in said at least one container having at least some longitudinal exposure; at least one evaporative surface device which is at least partially located in said at least one evaporative surface device opening and in said at least one fluid reservoir; wherein said at least one evaporative surface device is fluidly connected to said volatile material; and at least one leak trap located above said at least one reservoir.

In many of the cited records, a manipulation by the user is required with the risk of spilling the content of the containers.

On the other hand, the weight of the devices that comprise a container with the substance to be vaporized can overcome the resistance of the grid, making it impossible to direct the air stream at will.

It should not be forgotten that plastic consumption is high in most of the solutions found, which makes it necessary to look for solutions with lower plastic consumption that are more environmentally friendly.

### DESCRIPTION OF THE INVENTION

To solve the problems described above, an air freshener is proposed that incorporates an impregnated element that is exposed, at least perimetrally, to air currents, especially if it is configured to be fixed to a ventilation grid of a vehicle.

This air freshener has a minimal structure with the consequent saving of materials and, in addition, in a preferred execution in which its main elements have a cylindrical shape, it easily allows its manufacture in wood by means of a lathe, making the piece environmentally friendly.

To this end, the device comprises:
- A stem
- At least a first stop removable from the stem.
- A second stop that may be removable from the stem.
- One or more impregnated bodies arranged in the stem between the stops.

Optionally, a clamp with a suitable head to be inserted into the stem.

In a preferred embodiment, the device has a cylindrical shape and all its elements, except for the optional clamp, are coaxial, the device being structured around the physical axis defined by the stem.

In a preferred embodiment, the second stop and the stem are permanently joined, said second stop being located at an end of the stem.

In another possible embodiment, both stops are removable and the stem is independent and has anchoring means at its ends for joining the first stop and the second stop, closing the assembly.

The removable stop(s) is (are) attached to the stem by anchoring means. The anchoring facilitates the union, which can be produced by threading, by elastic adjustment or any other means that allows temporarily, but stably, fixing the stop to the stem, such that the user can undo and redo the union it repeatedly.

By elastic adjustment is meant any joint in which a deformable element is present, whether it is on the stop or on the stem, which generates pressure, friction or both in the areas of contact between the b stop and the stem.

Between the first stop and the second stop, there is a free stem section suitable for locating one or more impregnated bodies or, where appropriate, the clamp head.

In a preferred embodiment, the free section of the stem has an impregnated body and the clamp head.

The user can remove one of the stops and remove the impregnated body replacing it with another, incorporate another impregnated body or change the air freshener configuration by inserting or removing the clamp head. This operation will be common when the impregnated body has evaporated the substance that impregnates it and must be replaced by a soaked replacement.

### BRIEF DESCRIPTION OF THE FIGURES

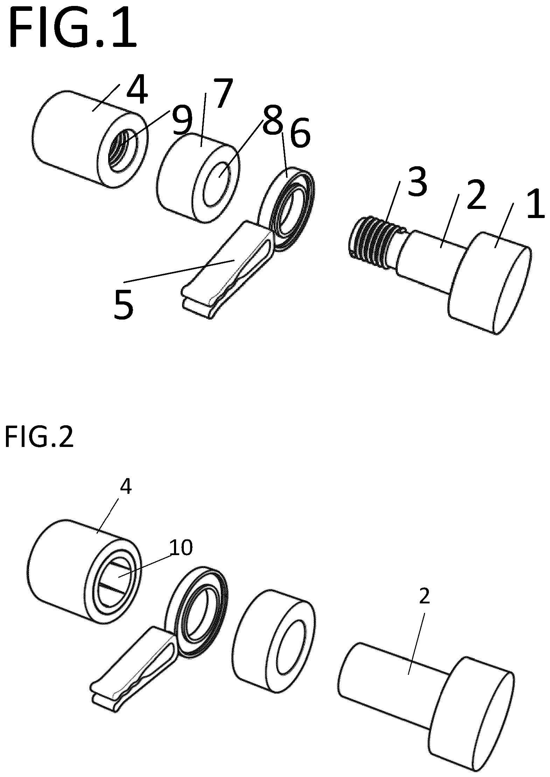

FIGURE 1 is an exploded view of the device showing the second stop (1), in this case joined to the stem (2), the threaded end (3) of the stem, the first stop (4), in this case the only removable one, with a hole joining the stem comprising an inner thread (9), the clamp (5) with an annular head (6) and the impregnated body (7) with a through hole (8) suitable for the stem to be inserted.
FIGURE 2 is an exploded view of the device showing an embodiment in which the union between the stem (2) and the first stop (4), in this case the only removable one, is by elastic adjustment, said first stop having an elastic reducer (10) in its hole for union to the stem.

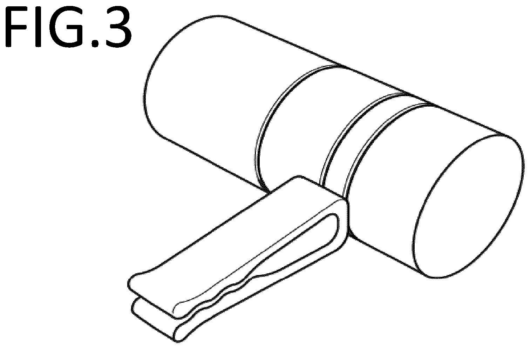

FIGURE 3 shows the device assembled and ready for use.

### DESCRIPTION OF A METHOD OF CARRYING OUT THE INVENTION

Described herein is a manner of carrying out the invention that is not unique but merely explanatory of the invention.

The invention relates to an air freshener device with a reduced and versatile structure in such a way that in one configuration the device can be fixed, for example, to the grid of the ventilation system of a car while in another configuration it can be arranged in any gap, barely occupying space.

For this purpose, the structure comprises a stem (2) that has attached to its ends a first stop (2) and a second stop (4), at least one of the two being removable, although in the embodiment described only the first stop (4) will be removable, and wherein the free section of the stem is suitable to accommodate an impregnated body (7) and an annular head (6) of a clamp (5).

The geometry of the structure of the air freshener, based on independent cylinders, allows its manufacture with a lathe, which facilitates the use of wood to replace plastics or other materials that are less environmentally friendly.

The device comprises:
a) A structure comprising the following coaxial elements;
   - A second fixed stop (1).
   - A stem (2) solidly attached to the second stop (1).
   - A first stop (4) removable with respect to the stem and attached to it by removable fixing means.
b) An impregnated body (7) inserted into the stem between the first stop (4) and the second stop (1).
c) A clamp (5) with an annular head (6) suitable for being inserted into the stem (2).

Both the first stop (4) and the second stop (1) and the stem (2) are cylindrical in shape and coaxially aligned.

The stem (2) and the first stop (4) are joined together and remain joined to each other with a removable joint, specifically by a thread (3) present at the outer end of the stem, said thread being complementary to an inner thread (9) present in the hole provided for joining the stem at the inner base of the first stop (4).

In another possible embodiment the first stop (4) and the inner stem (2) are joined and remain joined by means of a removable joint by elastic adjustment, since in the stem attachment hole provided in the first stop (4) a flexible reducer is arranged suitable for generating pressure and friction when the end of the stem is introduced.

When the second stop (4) is separated from the stem, an impregnated body (7) can be inserted into the stem, for which purpose it has a central hole (8), and the annular head (6) of the clamp (5).

Once the impregnated body (7) and the annular head (6) of the clamp (5) are arranged on the stem (2), the second stop (4) is fixed to the latter, thus preventing the impregnated body or the head of the clamp from leaving the stem.

The clamp, which comprises the annular head and clamping extensions, allows the device to be located in an air stream such as the ventilation grid of a car, thus favouring the dispersion of the perfume.

## Claims

1. AIR FRESHENER HAVING A STEM STRUCTURE WITH STOPS, **characterised in that** it comprises a stem (2) closed at its ends by a first stop (4) and a second stop (1), wherein at least the first stop (4) is removable and wherein the first stop, the second stop and the stem are coaxially aligned, and **in that** it comprises, arranged between both stops, an impregnated body (7) with a through hole (8) crossed by the stem.
Wherein the Air Freshener further comprises a clamp (5) with an annular head (6) said annular head being arranged between the first stop (4) and the second stop (1) and crossed by the stem.

2. AIR FRESHENER HAVING A STEM STRUCTURE WITH STOPS according to claim 1, **characterised in that** the first stop (2), the second stop (4) and the stem (2) are cylindrical.

3. AIR FRESHENER HAVING A STEM STRUCTURE WITH STOPS according to claim 1, **characterised in that** the first stop (2), the second stop (4) and the stem (2) are made of wood.

4. AIR FRESHENER HAVING A STEM STRUCTURE WITH STOPS according to claim 1, **characterised in that** the stem (2) and the at least one removable stop are joined by means of a thread (3) arranged at the end of the stem (2) and an inner thread (9) present in a hole for joining to the stem of the at least one removable stop.

5. AIR FRESHENER HAVING A STEM STRUCTURE WITH STOPS according to claim 1, **characterised in that** the stem (2) and the at least one removable buffer are joined by elastic adjustment, there being an elastic reducer (10) inside the hole for joining the stem to the at least one removable stop.

6. AIR FRESHENER HAVING A STEM STRUCTURE WITH STOPS according to claim 6, **characterised in that** the elastic reducer (10) comprises elastic materials.

7. AIR FRESHENER HAVING A STEM STRUCTURE WITH STOPS according to claim 6, **characterised in that** the elastic reducer (10) comprises cork.

## Patentansprüche

1. LUFTERFRISCHER MIT SCHAFTSTRUKTUR MIT ANSCHLÄGEN, **dadurch gekennzeichnet, dass** er einen Schaft (2) umfasst, der an seinen Enden durch einen ersten Anschlag (4) und einen zweiten Anschlag (1) verschlossen ist, wobei zumindest der erste Anschlag (4) abnehmbar ist und wobei der erste Anschlag, der zweite Anschlag und der Schaft koaxial ausgerichtet sind, und dass er zwischen beiden Anschlägen einen imprägnierten Körper (7) mit einem Durchgangsloch (8) umfasst, das vom Schaft gekreuzt wird.
Wobei der Lufterfrischer ferner eine Klemme (5) mit einem ringförmigen Kopf (6) umfasst, wobei der ringförmige Kopf zwischen dem ersten Anschlag (4) und dem zweiten Anschlag (1) angeordnet ist und vom Schaft gekreuzt wird.

2. LUFTERFRISCHER MIT SCHAFTSTRUKTUR MIT ANSCHLÄGEN nach Anspruch 1, **dadurch gekennzeichnet, dass** der erste Anschlag (2), der zweite Anschlag (4) und der Schaft (2) zylindrisch sind.

3. LUFTERFRISCHER MIT SCHAFTSTRUKTUR MIT ANSCHLÄGEN nach Anspruch 1, **dadurch gekennzeichnet, dass** der erste Anschlag (2), der zweite Anschlag (4) und der Schaft (2) aus Holz hergestellt sind.

4. LUFTERFRISCHER MIT SCHAFTSTRUKTUR MIT ANSCHLÄGEN nach Anspruch 1, **dadurch gekennzeichnet, dass** der Schaft (2) und der mindestens eine abnehmbare Anschlag mittels eines Gewindes (3), das am Ende des Schafts (2) angeordnet ist, und eines Innengewindes (9), das in einer Bohrung zum Verbinden mit dem Schaft des mindestens einen abnehmbaren Anschlags vorhanden ist.

5. LUFTERFRISCHER MIT SCHAFTSTRUKTUR MIT ANSCHLÄGEN nach Anspruch 1, **dadurch gekennzeichnet, dass** der Schaft (2) und der mindestens eine abnehmbare Puffer durch elastische Einstellung verbunden sind, wobei sich ein elastisches Reduzierstück (10) innerhalb der Bohrung befindet, um den Schaft mit dem mindestens einen abnehmbaren Anschlag zu verbinden.

6. LUFTERFRISCHER MIT SCHAFTSTRUKTUR MIT ANSCHLÄGEN nach Anspruch 6, **dadurch gekennzeichnet, dass** das elastische Reduzierstück (10) elastische Materialien umfasst.

7. LUFTERFRISCHER MIT SCHAFTSTRUKTUR MIT ANSCHLÄGEN nach Anspruch 6, **dadurch gekennzeichnet, dass** das elastische Reduzierstück (10) Kork umfasst.

## Revendications

1. DÉSODORISANT AYANT UNE STRUCTURE DE TIGE AVEC BUTÉES, **caractérisé en ce qu'**il comprend une tige (2) fermée à ses extrémités par une première butée (4) et une deuxième butée (1), au moins la première butée (4) étant amovible et la première butée, la deuxième butée et la tige étant alignées coaxialement, et **en ce qu'**il comprend, disposé entre les deux butées, un corps imprégné (7) avec un trou traversant (8) traversé par la tige.
Le désodorisant comprenant en outre une pince (5) avec une tête annulaire (6), ladite tête annulaire étant disposée entre la première butée (4) et la deuxième butée (1) et traversée par la tige.

2. DÉSODORISANT AYANT UNE STRUCTURE DE TIGE AVEC DES BUTÉES selon la revendication 1, **caractérisé en ce que** la première butée (2), la deuxième butée (4) et la tige (2) sont cylindriques.

3. DÉSODORISANT AYANT UNE STRUCTURE DE TIGE AVEC DES BUTÉES selon la revendication 1, **caractérisé en ce que** la première butée (2), la deuxième butée (4) et la tige (2) sont en bois.

4. DÉSODORISANT AYANT UNE STRUCTURE DE TIGE AVEC DES BUTÉES selon la revendication 1, **caractérisé en ce que** la tige (2) et l'au moins une butée amovible sont reliées au moyen d'un filetage (3) disposé à l'extrémité de la tige (2) et d'un filetage interne (9) présent dans un trou pour se relier à la tige de l'au moins une butée amovible.

5. DÉSODORISANT AYANT UNE STRUCTURE DE TIGE AVEC DES BUTÉES selon la revendication 1, **caractérisé en ce que** la tige (2) et l'au moins un tampon amovible sont reliés par réglage élastique, un réducteur élastique (10) étant situé à l'intérieur du trou pour relier la tige à l'au moins une butée amovible.

6. DÉSODORISANT AYANT UNE STRUCTURE DE TIGE AVEC DES BUTÉES selon la revendication 6, **caractérisé en ce que** le réducteur élastique (10) comprend des matériaux élastiques.

7. DÉSODORISANT AYANT UNE STRUCTURE DE TIGE AVEC DES BUTÉES selon la revendication 6, **caractérisé en ce que** le réducteur élastique (10) comprend du liège.
